(19) Europäisches Patentamt
European Patent Office
Office européen des brevets

(11) EP 2 543 401 B1

# (12) EUROPEAN PATENT SPECIFICATION

(45) Date of publication and mention of the grant of the patent:
**02.01.2019 Bulletin 2019/01**

(21) Application number: **11750630.3**

(22) Date of filing: **01.03.2011**

(51) Int Cl.:
***A61L 15/46*** *(2006.01)* ***A61L 26/00*** *(2006.01)*

(86) International application number:
**PCT/JP2011/054583**

(87) International publication number:
**WO 2011/108520 (09.09.2011 Gazette 2011/36)**

## (54) MATERIAL FOR SKIN, AND METHOD FOR PRODUCING MATERIAL FOR SKIN

MATERIAL FÜR DIE HAUT SOWIE VERFAHREN ZUR HERSTELLUNG DES MATERIALS FÜR DIE HAUT

PRODUIT POUR LA PEAU ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2010 JP 2010292169**
**02.03.2010 JP 2010045038**

(43) Date of publication of application:
**09.01.2013 Bulletin 2013/02**

(73) Proprietor: **Toray Industries, Inc.**
**Tokyo 103-8666 (JP)**

(72) Inventors:
- **KITAGAWA, Rumiko**
  **Otsu-shi**
  **Shiga 520-8558 (JP)**
- **NAKAMURA, Masataka**
  **Otsu-shi**
  **Shiga 520-8558 (JP)**
- **KUSUDA, Satoshi**
  **Tokyo 162-8666 (JP)**

(74) Representative: **Prüfer & Partner mbB**
**Patentanwälte · Rechtsanwälte**
**Sohnckestraße 12**
**81479 München (DE)**

(56) References cited:
**JP-A- 1 503 072**
**JP-A- 11 180 847**
**JP-A- 59 210 014**
**JP-A- 2008 083 649**
**JP-A- 2008 535 956**
**JP-A- 2009 148 392**
**JP-A- 2009 500 645**
**JP-A- 2010 032 992**
**JP-A- 2010 148 772**
**US-A- 4 991 574**
**US-A1- 2007 129 474**

EP 2 543 401 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

[Technical Field]

The present invention relates to a material for skin. The material for skin is a material which is used in skin protective materials for the purpose of coating or preventing wound, inflammation, bedsore and the like; sanitary goods such as sanitray items and paper diapers; various drug carriers such as medicines for external use (base material for applying to the skin); cosmetics such as materials for gel-like face mask; and cell culture media (cell proliferation media).

[Background Art]

A wound coating material and a skin protective material, which contain an antibacterial agent, are known. For example, Patent Literature 1 discloses a hydrogel therapeutic agent for the damaged skin, which contains, as a base material, native gellan gum containing povidone iodine which is an antibacterial agent. It is important to sufficiently spread oxygen all over the affected part to be coated such that the therapeutic agent for the damaged skin enables quick healing of the damaged skin. While this therapeutic agent for the damaged skin has high antibacterial properties, there was no saying that the therapeutic agent has high oxygen permeability.

Patent Literature 2 discloses a formulation for wound coating, which is prepared by adding, in addition to aqueous polymers such as polyacrylic acid and gellan gum as well as a gelling agent such as a polyvalent metal compound, optional components, for example, pharmaceutically active ingredients such as iodine and povidone iodine. However, these formulations for wound coating had not high oxygen permeability similarly to the formulation disclosed in Cited Literature 1.

Patent Literature 3 discloses a wound coating material which contains a polyether-based copolymer, a compound having a hydrosilyl group and a hydrosilylation catalyst, and can use povidone iodine as an optional component, and also exhibits satisfactory wettability and compatibility with the skin. It is considered that oxygen permeability is improved since such a material contains a compound having a hydrosilyl group. However, there was limitation on the amount of such a compound having a hydrosilyl group to be added.

Patent Literature 4 discloses a wound coating material which includes a three-layered structure of skin contact layer/absorption layer made of a polyether-based copolymer having a siloxane bond/backing layer and enables mixing of povidone iodine in an absorption layer and mixing of glycerin or povidone iodine in a skin contact layer, as optional components, and also causes less skin stimulation.

Patent literature US2007/129474 A1 discloses a material for skin contact comprising a polymer obtained from a silicone monomer and further comprises an antibacterial agent.

[Cited Literature List]

[Patent Literature]

[Patent Literature 1]
Japanese Unexamined Patent Publication (Kokai) No. 2002-104974
[Patent Literature 2]
Pamphlet of International Publication No. WO 2002/022182
[Patent Literature 3]
Japanese Unexamined Patent Publication (Kokai) No. 2009-148392
[Patent Literature 4]
Japanese Unexamined Patent Publication (Kokai) No. 2009-148393

[Summary of Invention]

[Technical Problem]

As mentioned above, the materials disclosed in Cited Literatures 1, 2 have no high oxygen permeability. According to the study of the inventors, it has been found that the material including a polyether-based copolymer disclosed in Patent Literature 3 has a problem that it is inferior in retention of povidone iodine. It is considered that the material disclosed in Patent Literature 4 also has a similar problem since it includes a polyether-based copolymer. There is also a problem that a polyether-based copolymer having a siloxane bond is inferior in shape retention when it contains an

alcohol.

An object of the present invention is to provide a material for skin, which is transparent and has a moderate moisture content, and also has high oxygen permeability, and a method for producing the same.

[Solution to Problem]

In order to achieve the above object, the present invention has the following constitutions.

The present invention is directed to a material for skin, as defined in claims 1-6.

The present invention also includes a method for producing a material for skin, as defined in claim 7.

[Advantageous Effects of Invention]

According to the present invention, it is possible to obtain a material for skin, which is transparent, and also has a moderate moisture content and high oxygen permeability. The material for skin of the present invention is suitably used in various medical devices, particularly a wound coating material.

The material for skin of the present invention is imparted with antibacterial properties by inclusion of an antibacterial agent, and thus infectious diseases can be reduced when used. The material for skin of the present invention is imparted with water retention by inclusion of an alcohol, and thus drying can be reduced when used. Furthermore, the material for skin of the present invention can be imparted with both antibacterial properties and water retention by inclusion of both an antibacterial agent and an alcohol.

[Description of Embodiments]

The present invention is a material for skin, including a silicone component-containing polymer obtained by polymerizing a silicone monomer having a carbon-carbon double bond, and a component selected from an antibacterial agent wherein the silicone monomer is a silicone monomer including no polyether structure, wherein the antibacterial agent contains halogen, and wherein the content of hydroxyl groups in the silicone monomer is from 0.0005 to 0.01 equivalent/gram.

In the present invention, the silicone monomer means.a compound having a siloxanyl group and a carbon-carbon double bond. The siloxanyl group means a group having at least one Si-O-Si bond.

The silicone monomer is preferably a silicone monomer which gives a polymer having excellent balance among water retention, oxygen permeability and transparency.

Specific examples of the structure which is suited as the silicone monomer include: silicone monomers of the following formulas (a) and (b):

[Chemical Formula 8]

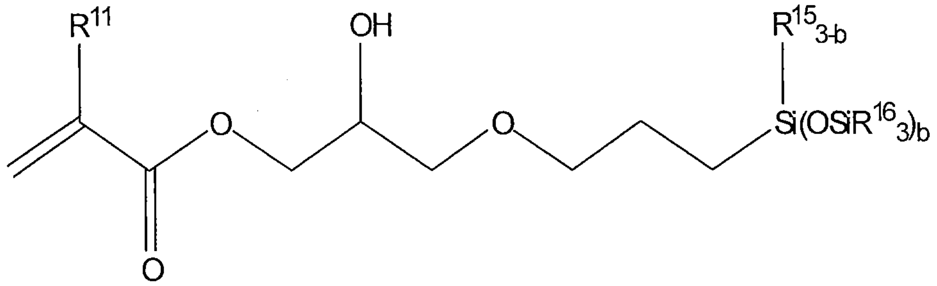

(a)

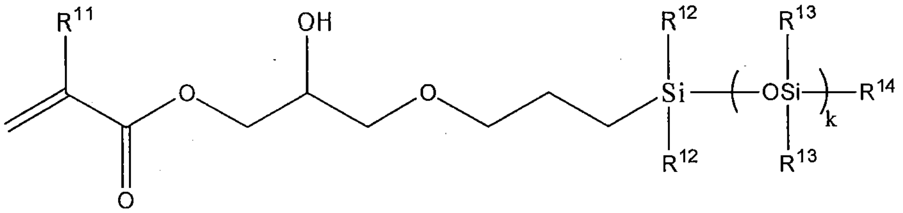

(b)

wherein k in the formulas (a) and (b) represents an integer of 0 to 100, b represents an integer of 1 to 3, $R^{11}$ represents H or a methyl group, $R^{12}$ and $R^{13}$ each independently represents a substituent selected from an alkyl group having 1 to 18 carbon atoms and a phenyl group, $R^{14}$ represents a substituent selected from an alkyl group having 1 to 6 carbon atoms and a phenyl group, and $R^{15}$ and $R^{16}$ each independently represents a substituent selected from an alkyl group

having 1 to 18 carbon atoms and a phenyl group.

In the present description, the term "(meth)acryloxy" means both methacryloxy and acryloxy, and the same shall apply to the terms "(meth)acryl" and "(meth)acryloyl".

The material for skin of the present invention contains a polymer prepared by polymerizing the above silicone monomer (silicone component-containing polymer). The detailed reason why such a silicone component-containing polymer is excellent in water absorbency and water retention, as compared with a polydimethylsiloxane (silicone rubber) compound which has hitherto been widely used in a material for skin, is unclear, but is estimated as follows. Namely, the polydimethylsiloxane compound and polydimethylsiloxane are the same in that a main chain skeleton of a polymer is formed by a siloxane bond and does not have carbon-carbon bond, whereas, the silicon component-containing polymer according to the present invention has a polysiloxane site and a fixed moiety of a main chain skeleton is formed from a carbon-carbon bond. It is considered that the silicon component-containing polymer according to the present invention is excellent in water absorbency and water retention as compared with the polydimethylsiloxane compound due to a difference in polymer structure. Accordingly, it is estimated that, when dipped in an aqueous solution of an antibacterial agent or an alcohol, absorbency and retention of the antibacterial agent or alcohol are higher than those of a conventional polydimethylsiloxane compound.

The inventors have also found that, when such a monomer includes a polyether structure, retention of an antibacterial agent such as povidone iodine in a material for skin deteriorates . Furthermore, the inventors have found that, when water retention is imparted to the material for skin using polyethylene glycol, shape retention of the material for skin is inferior since polyethylene glycol probably has high molecular mobility. Namely, the silicone monomer of the present invention is preferably a silicone monomer which has a carbon-carbon double bond and also includes no polyether structure.

In the present invention, the polyether structure means a structure in which a structural unit containing an ether bond represented by the general formula (-R-O-) is repeated. Herein, -R- is a divalent alkylene group. Specific examples of the polyether structure include a polyethylene oxide structure, a polypropylene oxide structure and the like. The above polyether structure may be composed of either one kind of a repeating unit, or two or more kinds of repeating units. Furthermore, the polyether structure may be either a linear structure or a branched structure.

From the above viewpoint, examples of more preferred compound as the silicone monomer used in the present invention include (a) and (b) since a silicone monomer having a (meth) acryloyl group is excellent in polymerizability.

A group having a carbon-carbon double bond in the silicone monomer is preferably a (meth) acryloyl group because of excellent polymerizablity of the monomer, and most preferably a methacryloyl group because of excellent chemical stability of the material for skin.

Too small number of silicon atoms of a siloxanyl group included in a molecule of a silicone monomer is not preferred since it becomes difficult to impart sufficient oxygen permeability to the material for skin. In contrast, too large number of silicon atoms of a siloxanyl group is not preferred since hydrophobicity of the material for skin becomes strong, and thus it becomes difficult to reconcile hydrous property and transparency. The number of silicon atoms is more preferably from 2 to 31, still more preferably from 2 to 21, and most preferably from 3 to 11.

The material for skin of the present invention preferably contains silicon atoms in the amount of 5 to 30% by weight based on the dry weight. Herein, the content of silicon atoms based on the dry weight of the material for skin can be measured by inductively coupled plasma (ICP) atomic emission spectrometer (preferably a sequential ICP atomic emission spectrometer SPS4,000, manufactured by Seiko Instruments Inc.). The measurement method is as follows.

First, a material for skin is set up in a dry state. In the present invention, the dry state means a state where a material for skin is subjected to vacuum drying at 40°C for 16 hours. The vacuum degree in the vacuum drying is adjusted to 2 hPa or less. After weighing a material for skin (4 to 5 mg) in a dry state in a platinum crucible, sulfuric acid is added and the material was incinerated by heating using a hot plate and a burner. The obtained lime is fused with sodium carbonate and dissolved under heating by adding water, and then nitric acid is acid and made to a constant volume with water. With respect to this solution, a silicon atom is measured by ICP atomic emission spectrometry thereby to determine the content of silicon atoms in the material for skin.

When the content of silicon atoms in the material for skin is too small, sufficient oxygen permeability cannot be obtained. When the content of silicon atoms is too large, it becomes impossible to obtain a transparent material for skin. From such a viewpoint, the content is preferably from 5 to 30% by weight, more preferably from 10 to 25% by weight, and most preferably from 10 to 20% by weight.

The silicone monomer is a silicone monomer having a hydroxyl group. A silicone component-containing polymer obtained by polymerizing the silicone monomer having a hydroxyl group is preferable since it is possible to obtain a polymer which is transparent in a hydrous state even when the below-mentioned internal wetting agent is included. If the polymer is transparent in a hydrous state, it is possible to observe the skin even in an applied state when used, for example, as a wound coating material or a skin protective material. Since it is not easy to obtain a transparent material for skin even when the content of hydroxyl groups in a silicone monomer is too small or large, at least one kind of a silicone monomer contains 0.0005 to 0.01 equivalent/g, more preferably 0.0008 to 0.008 equivalent/g and most preferably

0.001 to 0.005 equivalent/g, of hydroxyl groups. The content of hydroxyl groups in the silicone monomer can be specified by identifying a structure of a silicone monomer having a hydroxyl group using various analysis methods such as gas chromatograph mass spectrometry (GC-MS), high-performance liquid chromatograph mass spectrometry (HPLC-MS), nuclear magnetic resonance (NMR) and infrared spectroscopy (IR).

Suitable examples of particularly preferred silicone monomer include silicone monomers of the following formulas (a) and (b):

[Chemical Formula 9]

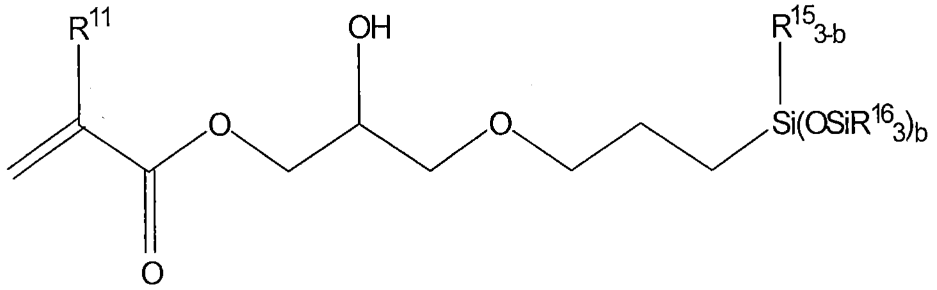

(a)

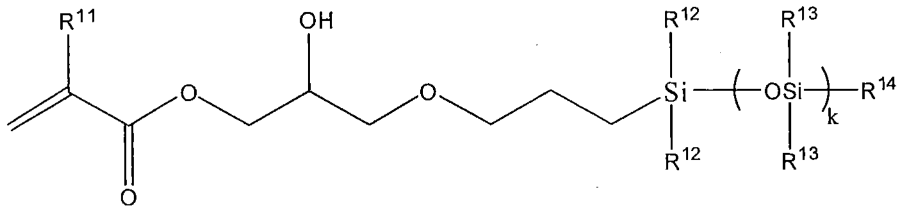

(b)

wherein k in the formulas (a) and (b) represents an integer of 0 to 100, b represents an integer of 1 to 3, $R^{11}$ represents H or a methyl group, $R^{12}$ and $R^{13}$ each independently represents a substituent selected from an alkyl group having 1 to 18 carbon atoms and a phenyl group, $R^{14}$ represents a substituent selected from an alkyl group having 1 to 6 carbon atoms and a phenyl group, and $R^{15}$ and $R^{16}$ each independently represents a substituent selected from an alkyl group having 1 to 18 carbon atoms and a phenyl group.

Since oxygen permeability of the obtained material for skin deteriorates when k is too small, and it becomes difficult to obtain a transparent material for skin when k is too small, k is more preferably from 1 to 30, still more preferably from 1 to 20, and most preferably from 2 to 10. Since oxygen permeability of the material for skin may deteriorate when b is 1, b is preferably 2 or 3. When b is more than 3, it may be sometimes difficult to obtain a transparent material for skin.

$R^{11}$ is preferably a methyl group from the viewpoint of chemical stability of the obtained material for skin.

$R^{12}$ and $R^{13}$ are preferably an alkyl group having 1 to 4 carbon atoms from the viewpoint of obtaining a polymer having high oxygen permeability, and most preferably a methyl group.

$R^{14}$ is preferably an alkyl group having 1 to 4 carbon atoms from the viewpoint of obtaining a polymer having high oxygen permeability, and most preferably a methyl group or a butyl group taking ease of production into consideration.

$R^{15}$ and $R^{16}$ are preferably an alkyl group having 1 to 4 carbon atoms from the viewpoint of obtaining a polymer having high oxygen permeability, and most preferably a methyl group taking ease of production into consideration.

The silicone component-containing polymer may be obtained by copolymerizing a monomer other than the silicone monomer. Examples of the other monomer in the case of copolymerization include monomers such as a hydrophilic monomer, a crosslinking agent, an ultraviolet absorber and a dye. A polymerizable group of the other monomer is preferably a (meth)acryloyl group, a styryl group, an allyl group and a vinyl group.

Examples of the other monomer are as follows: carboxylic acids such as (meth)acrylic acid, itaconic acid, crotonic acid and vinylbenzoic acid; alkyl (meth) acrylates such as methyl (meth) acrylate; (meth) acrylates having a hydroxyl group, such as 2-hydroxyethyl (meth)acrylate and glycerol (meth)acrylate; N-vinylcarboxylic acid amides such as N-vinylpyrrolidone, N-vinylformamide and N-vinyl-N-methylacetamide; (meth)acrylamides such as N,N-dimethylacrylamide; and aromatic vinyl monomers such as styrene. Among these monomers, a monomer having a (meth)acryloyl group is preferred from the viewpoint of easily obtaining a transparent material for skin.

From the viewpoint of easily obtaining a transparent material for skin, it is particularly preferred to copolymerize a (meth) acryloyl group-containingmonomer having a hydroxyl group, such as 2-hydroxyethyl (meth) acrylate. Since it is not easy to obtain the effect of improving transparency when the content of the (meth) acryloyl group-containing monomer having a hydroxyl group is too small, and an adverse influence is exerted on physical properties of a polymer when the content of the (meth) acryloyl group-containing monomer is too large, the content is preferably from 0.1 to

40% by weight, more preferably from 0.5 to 30% by weight, and most preferably from 1.0 to 20% by weight, based on the material for skin in a dry state.

From the viewpoint of an improvement in water retention, it is particularly preferred to copolymerize (meth) acrylamides such as N,N-dimethylacrylamide. Since it is not easy to obtain the effect of improving water retention when the content of the (meth)acrylamide group-containing monomer is too small, and an adverse influence is exerted on physical properties of a polymer when the content of the (meth) acryloyl group-containing monomer is too large, the content is preferably from 0.1 to 40% by weight, more preferably from 0.5 to 30% by weight, and most preferably from 1.0 to 20% by weight, based on the material for skin in a dry state.

From the viewpoint of obtaining a material for skin, which has satisfactory mechanical properties, and satisfactory tolerance to an antiseptic solution and a wash, it is preferred to use a monomer having two or more polymerizable groups in a molecule as a copolymerization component. The content of the monomer having two or more polymerizable groups in a molecule is preferably from 0.1% by weight to 10% by weight, more preferably from 0.3% by weight to 5% by weight, and still more preferably from 0.5% by weight to 3% by weight, based on the material for skin in a dry state.

The material for skin of the present invention may further contain additives such as an ultraviolet absorber, a pigment, a colorant and various stabilizers.

The material for skin of the present invention is imparted with antibacterial properties by inclusion of an antibacterial agent, and thus infectious diseases can be reduced when used.

The antibacterial agent is preferably an antibacterial agent having biocompatibility. Herein, biocompatibility means a property which exhibits low toxicity to the living body of humans and mammals . It is preferred when the antibacterial agent also has a property which is likely to be compatibility with the living body.

The antibacterial agent has the effect of decreasing the number of at least one of bacteria, eumycetes and ameba, and suppressing an increase in the number thereof. Because of potent antibacterial activity and long time duration, an antibacterial agent containing halogen is used as antibacterial agents. Examples of the antibacterial agent containing halogen include a quaternary antibacterial agent containing chlorine such as a salt of chlorhexidine or benzalkonium chloride, bromine and iodine. From the viewpoints of excellent durability of antibacterial performance, less influence on the material for skin, and no change in shape and appearance, silver, silver ions, iodine and iodine ions are particularly preferred, and iodine and iodine ions are most preferred. From the viewpoint of ease of handling, povidone iodine is particularly preferred among an iodine source .

When antibacterial properties are imparted to the material for skin of the present invention, it is preferred that the material for skin contains an antibacterial agent enough to exhibit antibacterial properties. However, since an adverse influence on the living body is concerned when an antibacterial agent excessively seeps out from the material for skin, excessive seeping out of the antibacterial agent is not preferred.

When the antibacterial agent to be used is iodine, it is preferred to include the antibacterial agent to such a degree that yellowish brown coloration derived from iodine is visually observed since the material for skin exhibits higher antibacterial properties. Even when coloration is not recognized, exhibition of antibacterial properties can be expected when odor derived from iodine is present. Because of lesser influence on the living body, it is preferred that idodine does not excessively seep out from the material for skin into water when the material for skin is dipped in water. When the material for skin is dipped in water, it is not preferred that yellowish brown coloration arises to such an extent that water can be visually seen since excessive seeping out of iodine used in the living body is concerned.

The material for skin of the present invention is imparted with water retention by inclusion of an alcohol, and thus drying can be reduced when used. When drying occurs when used, a problem such as severe pain arises upon removal from the skin. However, drying is suppressed by imparting water retention, and thus these problems can be relieved or suppressed. Moreover, as mentioned above, when water retention is imparted by using a silicone monomer including a polyether structure, the material for skin is inferior in shape retention. In contrast, when water retention is imparted by inclusion of an alcohol, using a silicone monomer including no polyether structure, it is possible to prevent deterioration of shape retention of the material for skin.

When water retention is imparted to the material for skin, the alcohol to be used is preferably an alcohol having a boiling point of 150°C or higher from the viewpoint of high water retention and high absorbency to the material for skin. The boiling point of the alcohol is more preferably 180°C or higher, and still more preferably 200°C or higher. An alcohol in which a boiling point does not exist under an atmospheric pressure is also very suitable. Among these alcohols, a polyhydric alcohol is preferred from the viewpoint of high water retention and less skin stimulation. Specific examples of the polyhydric alcohol in which a boiling point is 150°C or higher, or a boiling point does not exist under an atmospheric pressure, include ethylene glycol, diethylene glycol, dipropylene glycol, propylene glycol, polyethylene glycol, polypropylene glycol, butanediol, triethylene glycol, glycerin and the like. Among these polyhydric alcohols, glycerin and propylene glycol are particularly preferred from the viewpoint of high water retention and low skin stimulation, and glycerin is most preferably.

Furthermore, the material for skin of the present invention can be imparted with both antibacterial properties and water retention by inclusion of both an antibacterial agent and an alcohol. In this case, the antibacterial agent and

alcohol to be used are as follows. The case where the alcohol is glycerin and the antibacterial agent is iodine is most preferred.

The material for skin of the present invention may contain an ultraviolet absorber, a pigment, a colorant and the like. A silicone component-containing polymer may be copolymerized with an ultraviolet absorber having a polymerizable group, a pigment and a colorant.

The shape of the material for skin is preferably a sheet or a fluid. Herein, the sheet includes all planar shaped objects such as a plate, a film and a thin film. In the present description, conveniently, the plate may sometimes mean a material having a thickness of 15 mm or more, the film may sometimes mean a material having a thickness of 0.01 mm or more and less than 15 mm, and the thin film may sometimes mean a material having a thickness of less than 0.01 mm, respectively. The fluid may be any fluid in the form of wax, starch syrup, paste, oil, liquid and the like.

It is preferred that the material for skin is a sheet since the material can be used in contact with the skin by applying to the skin surface. It is not necessarily that the sheet has a complete two-dimensional shape, and may also have a three-dimensional shape (curve, etc.) which corresponds to a shape of the skin to which the sheet is applied. It is preferred that the material for skin is a fluid since the material can be used in contact with the skin by applying to the skin.

When the application of the material for skin is a skin protective material, a sheet-like form is particularly suitable. The thickness of the sheet is preferably 0.05 mm or more and less than 10 mm. Too small thickness is not preferred since the sheet is likely to be fractured because of poor strength. Too large thickness is not preferred since the sheet lacks conformity to skin movement and uncomfortable feeling of the applied site may increase. The area of the sheet is preferably from 0.1 $cm^2$ to 6,000 $cm^2$, more preferably from 0.2 $cm^2$ to 5,000 $cm^2$, and still more preferably from 1 $cm^2$ to 1,000 $cm^2$. Too small area of the sheet is not preferred since the effect of protecting the skin becomes insufficient. Too large area is not preferred since it becomes difficult to handle the sheet. From the viewpoint of ease of handling, a ratio of the length in a longitudinal direction (direction of the largest length) to the length of axial direction (direction of the smallest length) of the sheet is preferably 50 or less, more preferably 20 or less, and most preferably 10 or less. The shape of the sheet is preferably square, triangle, circle, ellipse and the like. It is suitably carried out that users cut a sheet into an appropriately desired shape when used.

Since it is possible to easily perform visual observation of skin conditions, the progress of a treatment and the like without removing the material for skin from the skin, the material for skin of the present invention is preferably transparent to such an extent that the opposite side can be visually observed through the material for skin. In particular, when the application of the material for skin is a skin protective material for the purpose of coating or preventing wound, inflammation, bedsore and the like, the skin protective material is preferably transparent.

It is preferred that the material for skin of the present invention has hydrous property. The moisture retention effect and body fluid absorption effect can be obtained by having hydrous property. An initial moisture content of the material for skin is preferably from 10 to 80% by weight, more preferably from 20 to 70% by weight, and most preferably from 30 to 60% by weight. Too low initial moisture content is not preferred since the moisture retention effect and body fluid absorption effect are not sufficiently obtained because of low hydrous property of the material for skin. Too high initial moisture content is not preferred since a shrinkage ratio of the material for skin may increase. The initial moisture content means moisture content in an initial state of the material for skin. Herein, the initial state means a state at the starting point of use when the material for skin is used as a product, or a state of a sample when subjected to the same conditions. Commonly, the initial state of each product is described or defined by a product description. As a general rule, immediately after removing a material for skin from a package as a product, the material for skin is washed by sequentially dipping in three water baths, each containing clean water, for 2 seconds each without a moment's pause, and then surface moisture is slightly wiped off. This state is regarded as the initial state. The initial state of samples produced in Examples of the present description will be described in the respective Examples.

As described in detailed below, the initial moisture content is determined by the equation below after measuring a weight (W1) in an initial state and a weight (W2) in a dry state of a material for skin.

$$\text{Initial moisture content (\%)} = (W1 - W2)/W1 \times 100$$

Too large shrinkage ratio of the material for skin is not preferred since deformation may arise and it may become impossible to coat the skin. The shrinkage ratio is preferably 20% or less, more preferably 15% or less, still more preferably 10% or less, and most preferably 8% or less.

The shrinkage ratio of the material for skin is determined by the following procedure . The length of a predetermined portion of the material for skin is measured in an initial state, and a state where the material for skin was stored for 48 hours in a desiccator at a temperature of 37°C and a humidity of 90%. The former length is designated as L1 and the latter length is designated as L2, thereby, the shrinkage ratio is determined by the equation below. Herein, the initial state is as defined above.

$$\text{Shrinkage ratio (\%)} = (L1 - L2)/L1 \times 100$$

When the material for skin has a square shape, each length of four sides is measured and an average of the shrinkage ratios to be calculated therefrom is regarded as shrinkage ratio. When the material for skin has a shape other than the square shape, (1) the length in a longitudinal direction (direction of the largest length) and (2) the length of the portion where the length becomes the largest in a direction perpendicularly intersected to the longitudinal direction are respectively measured, and then an average of the shrinkage ratios to be calculated therefrom is regarded as shrinkage ratio.

When the application is a lens for eye, the oxygen permeability of the material for skin of the present invention is preferably $70 \times 10^{-11}$ ($cm^2$/sec) $mLO_2$/(mL·hPa) or more from the viewpoint of the grade. The oxygen permeability coefficient is measured in water at 35°C using an oxygen permeability meter (Seikaken-type film-oxygen permeameter, manufactured by Rikaseiki Kogyo K. K.). The measurement is carried out in accordance with the method disclosed in columns 293 to 294 of the specification of U.S. Patent Application No. 2008/0081894.

A method for producing a material for skin of the present invention will be described below. A silicone component-containing polymer to be used in the material for skin of the present invention can be obtained by polymerizing the silicone monomer. At the time of the polymerization, a thermal polymerization initiator or a photopolymerization initiator, typified by a peroxide or an azo compound, is preferably added so as to facilitate the polymerization. In case thermal polymerization is performed, an initiator having optimum decomposition characteristics at a desired reaction temperature is selected when used. Commonly, an azo-based initiator and a peroxide-based initiator, each having a ten-hour half-life temperature of 40 to 120°C, are suitable. Examples of the photo polymerization initiator include a carbonyl compound, a peroxide, an azo compound, a sulfur compound, a halogen compound, a metal salt and the like. These polymerization initiators can be used alone or in combination. The polymerization initiator is preferably added in the amount of 1% by weight or less in a polymerization raw solution.

At the time of the polymerization, a polymerization solvent can be used. Various organic and inorganic solvents can be applied as the solvent. Examples thereof include water; various alcohol-based solvents such as methanol, ethanol, propanol, 2-propanol, butanol, tert-butanol and tert-amyl alcohol; various aromatic hydrocarbon-based solvents such as benzene, toluene and xylene; various aliphatic hydrocarbon-based solvents such as hexane, heptane, octane, decane, petroleum ether, kerosene, ligroin and paraffin; various ketone-based solvents such as acetone, methyl ethyl ketone and methyl isobutyl ketone; various ester-based solvents such as ethyl acetate, butyl acetate, methyl benzoate, dioctyl phthalate and ethylene glycoldiacetate; and various glycol ether-based solvents such as diethylether, tetrahydrofuran, dioxane, ethylene glycol dialkyl ether, diethylene glycol dialkyl ether, triethylene glycol dialkyl ether, tetraethylene glycol dialkyl ether, polyethylene glycol dialkyl ether, polyethylene glycol-polypropylene glycol block copolymer and polyethylene glycol-polypropylene glycol random copolymer. These solvents can be used alone or in combination. Among these solvents, alcohol-based solvents and glycol ether-based solvents are preferred from the viewpoint of easily removing the solvent from the obtained material for skin by washing with water.

At the time of the polymerization, it is preferred to add a hydrophilic polymer as an internal wetting agent in a polymerization raw solution so as to impart water wettability to a surface of a silicone component-containing polymer. Suitable examples of the hydrophilic polymer include polyvinylpyrrolidone, poly-N-vinyl-2-piperidone, poly-N-vinyl-2-caprolactam, poly-N-vinyl-3-methyl-2-caprolactam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2-pyrrolidone, and poly-N-vinyl-4,5-dimethyl-2-pyrrolidone, polyvinylimidazole, poly-N,N-dimethylacrylamide, polyvinyl alcohol, polyacrylic acid, polyethylene oxide, poly 2-ethyloxazoline, heparin polysaccharide, polysaccharide, and a mixture, a copolymer and a macromonomer thereof. Among these hydrophilic polymers, polyvinylpyrrolidone, poly-N,N-dimethylacrylamide, polyacrylic acid and polyvinyl alcohol are more preferred from the viewpoint of exhibiting satisfactory wettability. From the viewpoint of having satisfactory solubility in a polymerization raw solution, polyvinylpyrrolidone and poly-N,N-dimethylacrylamide are most preferred.

When the addition amount of the hydrophilic polymer is too small, sufficient wettability cannot be obtained and, when the addition amount of the hydrophilic polymer is too large, solubility in a polymerization raw solution containing a silicone prepolymer decreases. Therefore, the addition amount of the hydrophilic polymer is preferably from 0.1 to 30% by weight, more preferably from 0.5 to 25% by weight, and most preferably from 1 to 20% by weight, based on the polymerization raw solution. The addition amount of the hydrophilic polymer is preferably from 0.08 to 24% by weight, more preferably from 0.4 to 20% by weight, and most preferably from 0.8 to 16% by weight, based on the material for skin in a dry state.

When the molecular weight of the hydrophilic polymer is too small, sufficient wettability cannot be obtained and, when the amount of the molecular weight of the hydrophilic polymer is too large, the viscosity of a polymerization raw solution becomes too high. Therefore, the molecular weight of the hydrophilic polymer is preferably from 1,000 to

2, 000, 000, more preferably from 10,000 to 1,000,000, and most preferably from 200,000 to 800,000.

When applications of the material for skin are a sheet-like wound coating material and a skin protective material, examples of the polymerization method and molding method include the following methods.

Two hard plates (for example, glass plate), each having the size corresponding to the size of the objective material for skin, are prepared. An aluminum seal may be applied to the plate such that the obtained material for skin is easily removed. A gasket (which is obtained, for example, by cutting out the center portion of a parafilm) having a thickness corresponding to the thickness of the objective material for skin is interposed between two plates as a spacer. Vacant space formed by the plates and spacer is filled with a monomer, and photopolymerization or thermal polymerization is carried out to obtain a sheet-like material for skin. In the case of the photopolymerization, glass plates, vacant space of which is filled with a monomer, is irradiated with active rays such as ultraviolet ray. In the case of thermal polymerization, glass plates, vacant space of which is filled with a monomer, are heated by placing in an oven or a liquid bath. There can be a method using both methods in combination, in which thermal polymerization is carried out after the photopolymerization, or the photopolymerization is carried out after thermal polymerization. In the case of the photopolymerization, for example, it is common to be irradiated with light containing large quantities of ultraviolet ray from a mercury lamp or an insect trap lamp as a light source for a short time (usually 1 hours or less) . When thermal polymerization is carried out, there is employed favorite condition in which a temperature is gradually raised from about room temperature and is raised up to a temperature of 60°C to 200°C over several hours to several tens of hours so as to keep optical uniformity and grade of a polymer and to enhance reproducibility.

The material for skin of the present invention can be subjected to a modification treatment by various methods. When the application is a wound coating material, it is preferred to carry out a modification treatment for improving water wettability of a surface.

Specific modification method includes chemical vapor deposition treatments such as electromagnetic wave (including light) irradiation, plasma irradiation, vapor deposition and sputtering, a base treatment, an acid treatment, use of other appropriate surface treating agent, and a combination thereof. Among these modification means, a base treatment and an acid treatment are simple and preferred.

It is possible to exemplify, as an example of base and acid treatments, a method in which a material for skin is brought into contact with a basic or acidic solution, a method in which a material for skin is brought into contact with a basic or acidic gas. Specific method includes, for example, a method in which a material for skin is dipped in a basic or acidic solution, a method in which a basic or acidic solution or a basic or acidic gas is sprayed over a material for skin, a method in which a basic or acidic solution is applied to a material for skin using a spatula, a brush or the like, and a method in which a material for skin is subjected to a surface treatment by a spin coating method or a dip coating method using a basic or acidic solution. The method capable of obtaining large modification effect most simply is a method in which a material for skin is dipped in a basic or acidic solution.

There is no particular limitation on the temperature at which the material for skin is dipped in a basic or acidic solution, and the temperature is preferably within a range from about -50°C to 300°C. Taking workability into consideration, the temperature is more preferably within a range from -10°C to 150°C, and most preferably from -5°C to 60°C.

Regarding the time during which the material for skin is dipped in a basic or acidic solution, an optimum time varies depending on the temperature. Commonly, the optimum time is preferably within 100 hours, more preferably within 24 hours, and most preferably within 12 hours. When the contact time is too long, not only workability and productivity deteriorate, but also adverse influence such as deterioration of oxygen permeability and deterioration of mechanical properties may be sometimes exerted.

It is possible to use, as a base, high molecular weight bases such as alkalimetal hydroxide, alkali earth metal hydroxide, various carbonates, various borates, various phosphates, ammonia, various ammonium salts, various amines and polyethyleneimine, and polyvinylamine. Among these bases, alkali metal hydroxide is most preferred because of its low price and large treatment effect.

It is possible to use, as an acid, various inorganic acids such as sulfuric acid, phosphoric acid, hydrochloric acid and nitric acid; various organic acids such as acetic acid, formic acid, benzoic acid and phenol; and various high molecular weight acids such as polyacrylic acid and polystyrenesulfonic acid. Among these acids, high molecular weight acids are most preferred because of large treatment effect and less adverse influence on other physical properties.

It is possible to use, as a solvent of a basic or acidic solution, various inorganic and organic solvents. Examples of the solvent include water; various alcohols such as methanol, ethanol, propanol, 2-propanol, butanol, ethylene glycol, diethylene glycol, triethylene glycol, tetraethylene glycol, polyethylene glycol and glycerin; various aromatic hydrocarbons such as benzene, toluene and xylene; various aliphatic hydrocarbons such as hexane, heptane, octane, decane, petroleum ether, kerosene, ligroin and paraffin; various ketones such as acetone, methyl ethyl ketone and methyl isobutyl ketone; various esters such as ethyl acetate, butyl acetate, methyl benzoate and dioctyl phthalate; various ethers such as diethylether, tetrahydrofuran, dioxane, ethylene glycol dialkyl ether, diethylene glycol dialkyl ether, triethylene glycol dialkyl ether, tetraethylene glycol dialkyl ether and polyethylene glycol dialkyl ether; various aprotic polar solvents such as dimethylformamide, dimethylacetamide, N-methyl-2-pyrrolidone, dimethylimidazolidinone, hexamethylphosphoric triamide and dimethyl sulfoxide; halogen-based solvents such as methylene chloride, chloroform, dichloroethane, trichloroethane and trichloroethylene; and flon-based solvents. Among these solvents, water is most preferred from the viewpoint of economy, simplicity of handling and chemical stability. It is also possible to use, as the solvent, a mixture of two or more kinds of substances.

The basic or acidic solution may contain components other than the basic or acidic substance and solvent.

After the base treatment or acid treatment, excess basic or acidic substance is preferably removed. It is possible to use, as a cleaning solvent, various inorganic and organic solvents mentioned above. Among the solvents, water is most preferred. It is also possible to use a mixture of two or more kinds of solvents. The cleaning solvent may contain components other than the solvent, for example, inorganic salts, a surfactant, a cleaning agent and the like.

The above-mentioned modification treatment may be applied to the entire material for skin, or may be applied to only a part of the material for skin, for example, only a surface. When the modification treatment is applied only to the surface, it is possible to improve only water wettability of the surface without largely changing properties of the entire material for skin.

As mentioned above, the material for skin of the present invention can be imparted with a property selected from antibacterial properties by inclusion of an antibacterial agent Such a material for skin is obtained by an antibacterial agent to a silicone component-containing polymer. The method will be described below.

When antibacterial properties are imparted in the material for skin of the present invention, various methods can be used as the method of imparting an antibacterial agent to a silicone component-containing polymer. Used is a method in which a silicone component-containing polymer is dipped in a solution containing an antibacterial agent. From the viewpoint of enabling exhibition of high antibacterial properties and simple operation, the method in which a silicone component-containing polymer is dipped in a solution containing an antibacterial agent is particularly preferred.

In the case of dipping a silicone component-containing polymer in a solution containing an antibacterial agent, when the dipping time is too short, the antibacterial agent is not adsorbed to a material for skin. When the dipping time is too long, an adverse influence is exerted on shape, appearance and the like of the material for skin, and thus the dipping time is preferably from 5 minutes to 30 minutes.

When the concentration of a solution containing an , antibacterial agent is too low, antibacterial properties are not exhibited. When the concentration of a solution containing an antibacterial agent is too high, solubility of the antibacterial agent in the solution, and handling properties deteriorate. Therefore, the concentration of a solution containing an antibacterial agent is preferably 0.001% by weight to 10% by weight. In particular, when the antibacterial agent is iodine, antibacterial activity is high and thus the concentration of the solution is preferably from 0.001% by weight to 1% by weight. Because of less adverse influence on mechanical strength of a skin protective material, the concentration of the solution is most preferably from 0.005% by weight to 0.3% by weight.

The solvent to be used in a solution containing an antibacterial agent may be any solvent as long as it is a solvent which can uniformly dissolve the antibacterial agent. Because of low stimulation, alcohols or water is/are preferred, and water is most preferred.

When the dipping temperature, at which a silicone component-containing polymer is dipped in a solution containing an antibacterial agent, is too low and too high, the content of the antibacterial agent in the material for skin is low and the silicone component-containing polymer is largely damaged. Therefore, the dipping temperature is preferably from -5°C to 100°C, more preferably from 0°C to 60°C, and most preferably from 10°C to 40°C.

When water retention is imparted in the material for skin of the present invention, it is possible to use various methods as the method of imparting an alcohol to the silicone component-containing polymer. Examples of the method include a method in which an alcohol is added at the time of silicone monomer polymerization, a method in which a silicone component-containing polymer is dipped in an alcohol or a solution containing an alcohol and the like. The method in which in which an alcohol is added at the time of silicone monomer polymerization is preferred from the viewpoint of enabling inclusion of a large amount of an alcohol in the material for skin. However, this method may sometimes fail to sufficiently exhibit high water retention. From the viewpoint of enabling exhibition of high water retention and simple operation, the method in which a silicone component-containing polymer is dipped in an alcohol or a solution containing an alcohol is particularly preferred.

When the dipping time, during which a silicone component-containing polymer is dipped in an alcohol or a solution containing an alcohol, is too short, the alcohols does not penetrate into a material for skin and, when the dipping time is too long, an adverse influence is exerted on shape, appearance and the like of a material for skin. Therefore, the dipping time is preferably from 24 hours to 96 hours. When a silicone component-containing polymer is dipped in an alcohol or a solution containing an alcohol, permeability of an alcohol into a material for skin is inferior even when the dipping temperature is too low and too high. When the dipping temperature is too high, a silicone component-containing polymer is largely damaged. Therefore, the dipping temperature is preferably from -5°C to 100°C, more preferably from 0°C to 60°C, and most preferably from 10°C to 40°C.

Since water retention of a material for skin deteriorates even when the concentration of a solution containing an alcohol is too low and too high, the concentration of a solution containing an alcohol is preferably from 20% by weight

to 80% by weight, and particularly preferably from 40 to 60% by weight. The solvent to be used in a solution containing an alcohol may be any solvent as long as it can uniformly dissolve an alcohol, and is preferably water because of low stimulation.

Even when the storage temperature after impairing water retention to a silicone component-containing polymer is too low and too high, damage on a silicone component-containing polymer is concerned, the storage temperature is preferably from -20°C to 100°C, more preferably from -10°C to 40°C, and most preferably from -5°C to 20°C. When the storage humidity is too low, damage on a silicone component-containing polymer is concerned and, when the storage humidity is too high, it is difficult to use since dew condensation of the storage area occurs. Therefore, relative humidity is preferably from 70% to 100%, more preferably from 75 to 95%, and most preferably from 80% to 90%.

In the material for skin of the present invention, when both antibacterial properties and water retention are imparted, it is possible to use various methods as a method of imparting an alcohol and an antibacterial agent to a silicone component-containing polymer. Examples of the method include a method in which a silicone component-containing polymer is dipped in an alcohol or a solution containing an alcohol, and then dipped in a solution containing an antibacterial agent; and a method in which a silicone component-containing polymer is dipped in an alcohol or a solution containing an alcohol, and then a solution containing an antibacterial agent is applied. The method in which a silicone component-containing polymer is dipped in an alcohol or a solution containing an alcohol, and then dipped in a solution containing an antibacterial agent is preferred from the viewpoint of exhibiting antibacterial properties. However, this method may sometimes fail to sufficiently exhibit high water retention. In order to enable a material for skin to exhibit high water retention and high antibacterial properties, it is preferred to use a method in which an alcohol is first imparted to a silicone component-containing polymer, and then a solution containing an antibacterial agent is applied on a surface of a silicone component-containing polymer containing an alcohol. From the viewpoint of a simple operation, particularly preferred method is a method in which a silicone component-containing polymer is dipped in an alcohol or a solution containing an alcohol, and then a solution containing an antibacterial agent is applied.

Herein, a solvent to be used in a solution containing an alcohol, the kind of an alcohol, the concentration of an alcohol, a temperature at which a material for skin is dipped in a solution containing an alcohol, a storage temperature after dipping, storage humidity, kind of an antibacterial agent, a solvent to be used in a solution containing an antibacterial agent, the concentration of a solution containing an antibacterial agent, and a temperature at which a material for skin is dipped in a solution containing an antibacterial agent are the same as above.

Namely, one of preferred aspects of the method for producing a material for skin of the present invention is directed to a method in which a silicone component-containing polymer obtained by polymerizing a silicone monomer composition having a carbon-carbon double bond is dipped in a solution containing a component selected from an antibacterial agent and an alcohol. Particularly preferred aspect of the case where both antibacterial properties and water retention are imparted to a material for skin is directed to a method in which a solution containing an antibacterial agent is applied on a surface of a material for skin, including a silicone component-containing polymer obtained by polymerizing a silicone monomer composition having a carbon-carbon double bond, and an alcohol.

The material for skin of the present invention is suited for use in skin protective materials for the purpose of coating or preventing wound, inflammation, bedsore and the like; sanitary items and paper diapers; and various drug carriers. The material for skin of the present invention exhibits moderate moisture content and high oxygen permeability, and also enables imparting of antibacterial properties, and thus causing less anxiety of infectious diseases and less adverse influence on the living body. Therefore, the material for skin is suited for use in a skin protective material for children, particularly infants, babies, newborn babies and the like. The material for skin is suited for use in a skin protective material for newborn babies, particularly premature infants born at less than 30 weeks of gestation, who have incompletely formed skin.

[Examples]

The present invention will be specifically described below by way of Examples, but the present invention is not limited thereto.

<Evaluation of Coloration>

When antibacterial properties are imparted, a material for skin was dipped in water for the below-mentioned days, and then visual observation was carried out and coloration was evaluated by the following criteria.

A: Uniform deep yellow coloration is observed in a material for skin, and no coloration is observed in water.
B: Uniform pale yellow coloration is observed in a material for skin, and no coloration is observed in water.
C: Uniform pale yellow coloration is observed in a material for skin, and pale yellow coloration is observed in water.
D: No coloration is observed in a material for skin, and deep yellow coloration is observed in water.

E: No coloration is observed in a material for skin, and also no coloration is observed in water.

<Evaluation of Water Retention>

A material for skin was stored in a desiccator for 24 hours, and then state observation was carried out by touching with an index finger and water retention was evaluated by the following criteria.

A: There is no difference in softness and degree of drying of a material for skin before and after storage.
B: After storage for 24 hours, hardness of a material for skin slightly increases as compared with the material for skin before storage, and drying is slightly observed.
C: After storage for 24 hours, hardness of a material for skin drastically increases as compared with the material for skin before storage, and drying sensation is high.

<Initial Moisture Content>

First, a weight (W1) of a material for skin in an initial state defined in the respective Examples and Comparative Examples was measured. Then, the material for skin was subjected to vacuum drying at 40°C for 16 hours and a weight (W2) in a dry state was measured, and initial moisture content was calculated by the following equation.

$$\text{Initial moisture content (\%)} = (W1 - W2)/W1 \times 100$$

<Shrinkage Ratio>

A length (L1) of a specific portion of a material for skin in an initial state defined in the respective Examples and Comparative Examples was measured. Then, the material for skin was stored in a desiccator at a temperature of 37°C and a humidity of 90% for 48 hours. A length (L2) of the portion corresponding to L1 of the material for skin after storage was measured. A shrinkage ratio was calculated by the following equation.

$$\text{Shrinkage ratio (\%)} = (L1 - L2)/L1 \times 100$$

When the material for skin has a square shape, each length of four sides was measured and an average of shrinkage ratios calculated from the length was regarded as a shrinkage ratio. When the material for skin has a lens shape, each diameter in two perpendicular intersecting directions was measured and an average of shrinkage ratios calculated from the diameters was regarded as a shrinkage ratio.

Example 1

A silicone monomer (13.4 parts by weight, hydroxyl group content: 0.0032 equivalent/g) represented by the following formula (c):

[Chemical Formula 10]

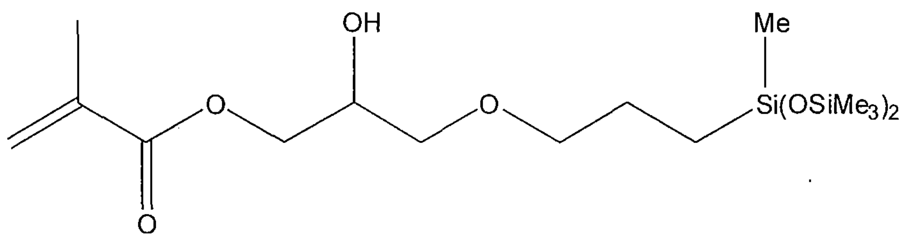

(c)

, N,N-dimethylacrylamide (28.0 parts by weight), a silicone monomer (36.6 parts by weight, hydroxyl group content: 0.006 equivalent/g) represented by the following formula (d):

[Chemical Formula 11]

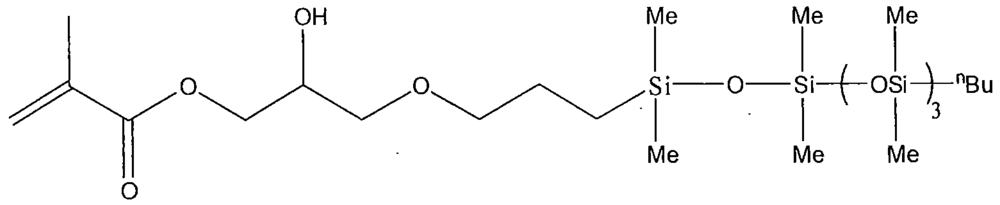

(d)

polyvinylpyrrolidone (molecular weight about 500,000, 12.0 parts by weight), a photoinitiator IRGACURE 1850 (1.0 parts by weight), methacrylic acid-2-hydroxyethyl (7.0 parts by weight), triethylene glycol dimethacrylate (1.0 parts by weight), a dye monomer (0.02 part by weight) represented by the following formula (e) :

[Chemical Formula 12]

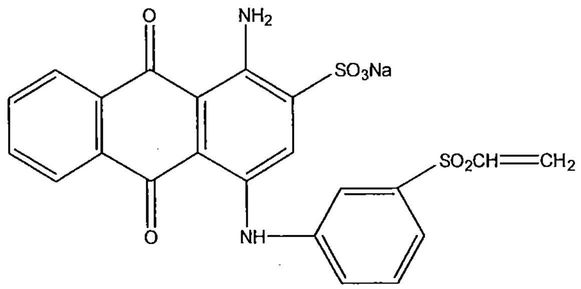

(e)

and tetrahydrolinalool (32.0 parts by weight) were mixed and then stirred. Herein, the compound of the formula (c) belongs to the compound represented by the formula (a), and the compound of the formula (d) belongs to the compound represented by the formula (b). As a result, a uniform and transparent monomer mixture was obtained. The obtained monomer mixture was degassed under an argon atmosphere.

In a glove box under a nitrogen atmosphere, two spacers obtained by cutting out a center portion of a 100 μm thick parafilm was interposed between two glass plates, each measuring 10 cm square and 3 mm in thickness (an aluminum seal is applied to one of glass plates). The vacant space formed by these glass plates and spacers was filled with the monomer mixture, which was cured by light irradiation (Toshiba FL6D, 8.4 kilolux for 20 minutes) to obtain a sheet-like film. The obtained film was dipped in an aqueous 60% by weight isopropanol (IPA) solution at 60°C for 30 minutes, removed from the glass plate and then dipped in an aqueous 80% by weight IPA solution at 60°C for 2 hours thereby to extract impurities such as a residual monomer. The film was dipped in a solution in which the concentration of IPA decreases in a stepwise manner, for example, an aqueous 50% by weight IPA solution, an aqueous 25% by weight IPA solution and the like for 30 minutes each, and finally dipped in water for 2 hours or more water thereby allowing to undergo hydration. Then, the film was dipped in a UM sample bottle containing water therein and was put in an autoclave, together with the UM sample bottle, and then a boiling treatment was carried out at 120°C for 30 minutes. After the boiling treatment, the film was washed with ultrapure water and then dipped in an "ISODINE" (registered trademark) solution (for gargle, Meiji Seika Kaisha, Limited) containing 7% by weight of povidone iodine for 15 minutes. Subsequently, the film was washed by dipping in three beakers each containing clean water (200 mL each) therein without a moment's pause for 2 seconds each, and then surface moisture was slightly wiped off by a gauze. A state immediately thereafter was regarded as an initial state, and the film was subjected to the measurement of the initial moisture content and shrinkage ratio.

The thus obtained film was stored in fresh water for 5 days. After storage for 1 day, coloration was evaluated as "A" and, after storage for 5 days, coloration was evaluated as "A". After storage for 5 days, uniform yellow coloration derived from povidone iodine was observed in the film, similar to the case after storage for 1 day. Immediately after dipping in an "ISODINE" (registered trademark) solution, after storage for 1 day and after storage for 5 days, the opposite side could be visually observed in all films and the films were transparent. The initial moisture content was 55% by weight and the shrinkage ratio was 20%. The content of silicon atoms of the obtained film was 11.1% by weight based on the dry weight and the oxygen permeability was 53 × $10^{-11}$ ($cm^2$/sec) $mLO_2$/(mL·hPa), and the film was suited for use as a material for skin.

Example 2

In the same manner as in Example 1, except that polydimethylacrylamide (molecular weight about 500,000, 12.0 parts by weight) was used in place of polyvinylpyrrolidone in Example 1, a film was produced. After storage for 1 day, coloration in water was evaluated as "A" and, after storage for 5 days, coloration in water was evaluated as "A". After storage for 5 days, uniform yellow coloration derived from povidone iodine was observed in the film, similar to the case after storage for 1 day. Immediately after dipping in an "ISODINE" (registered trademark) solution, after storage for 1 day and after storage for 5 days, the opposite side could be visually observed through the film in all films, and the films were transparent. The initial moisture content was 56% by weight, and the shrinkage ratio was 20%. The content of silicon atoms of the obtained film was 11.1% by weight based on the dry weight, and the shrinkage ratio was 53 $\times 10^{-11}$ ($cm^2$/sec) $mLO_2$/(mL·hPa) based on the dry weight, and the film was suited for use as a material for skin.

Example 3

Using a silicone monomer (13.4 parts by weight, hydroxyl group content: 0.0032 equivalent/g) represented by the above formula (c), N,N-dimethylacrylamide (37.0 parts by weight), a silicone monomer (36.6 parts by weight, hydroxyl group content: 0.006 equivalent/g) represented by the above formula (d), a photoinitiator IRGACURE 1850 (1.26 parts by weight), methacrylic acid-2-hydroxyethyl (9.2 parts by weight), triethylene glycol dimethacrylate (1.26 parts by weight), a dye monomer (0.02 part by weight) represented by the above formula (e), and tetrahydrolinalool (23.9 parts by weight), films were produced and evaluated in the same manner as in Example 1. After storage for 1 day, coloration in water was evaluated as "A" and, after storage for 5 days, coloration in water was evaluated as "A". After storage for 5 days, uniform yellow coloration derived from povidone iodine was observed in the film, similar to the case after storage for 1 day. Immediately after dipping in an "ISODINE" (registered trademark) solution, after storage for 1 day and after storage for 5 days, the opposite side could be visually observed through the film in all films, and the films were transparent. The initial moisture content was 60% by weight, and the shrinkage ratio was 20%. The content of silicon atoms of the obtained film was 11.1% by weight, and the shrinkage ratio was 53 $\times 10^{-11}$ ($cm^2$/sec) $mLO_2$/(mL·hPa), and the film was suited for use as a material for skin.

Example 4

Using a commercially available soft contact lens "Acuvue Advance" (register trademark) (manufactured by Johnson & Johnson Vision Care Inc., hereinafter referred to as J&J) produced by using, as a silicone monomer, the compounds including no polyether structure of the following formulas (f) and (g), the commercially available soft contact lens was dipped in an "ISODINE" (registered trademark) solution for 15 minutes and then stored in fresh water for 5 days in the same manner as in Example 1. After storage for 1 day, coloration in water was evaluated as "A" and, after storage for 5 days, coloration in water was evaluated as "A". After storage for 5 days, uniform yellow coloration derived from povidone iodine was observed in the film, similar to the case after storage for 1 day. Immediately after dipping in an "ISODINE" (registered trademark) solution, after storage for 1 day and after storage for 5 days, the opposite side could be visually observed through the film in all films, and the films were transparent. The initial moisture content was 45.9% by weight, and the shrinkage ratio was 17%.

[Chemical Formula 13]

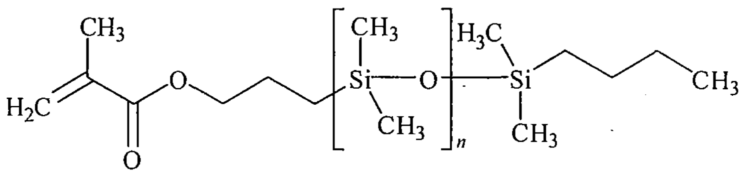

(f)

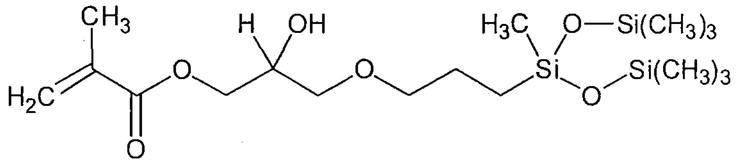

(g)

Example 5

Using a commercially available soft contact lens "Pure Vision" (register trademark) (manufactured by Bausch & Lomb, Inc., hereinafter referred to as B&L) produced by using, as a silicone monomer, the compounds including no polyether structure of the following formulas (h) and (i), the commercially available soft contact lens was dipped in an "ISODINE" (registered trademark) solution for 15 minutes and then stored in fresh water for 5 days in the same manner as in Example 1. After storage for 1 day, coloration in water was evaluated as "A" and, after storage for 5 days, coloration in water was evaluated as "A". After storage for 5 days, uniform yellow coloration derived from povidone iodine was observed in the film, similar to the case after storage for 1 day. Immediately after dipping in an "ISODINE" (registered trademark) solution, after storage for 1 day and after storage for 5 days, the opposite side could be visually observed through the film in all films, and the films were transparent. The initial moisture content was 33.4% by weight, and the shrinkage ratio was 12%.

[Chemical Formula 14]

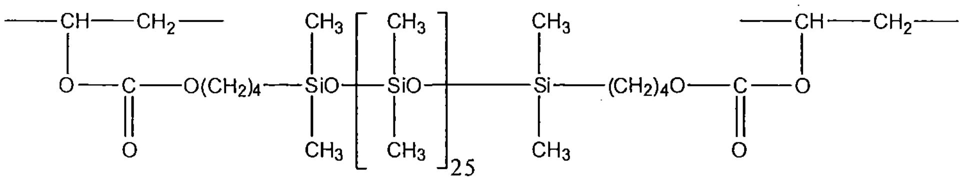

(h)

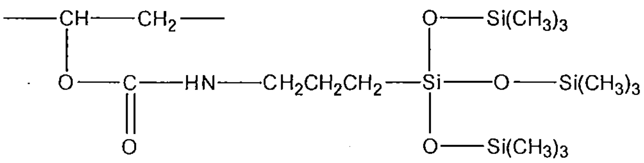

(i)

Example 6

The film produced in the same manner as in Example 1, except that dipping in the "ISODINE" (registered trademark) solution was not performed, was dipped in an aqueous 50% by weight glycerin solution for 72 hours (glycerin has a boiling point of 290°C) . Then, the film was sequentially dipped in three water baths, each containing clean water (200 mL each), for 2 seconds each without a moment's pause, and then surface moisture of the film was slightly wiped off with "Kimwipe" (register trademark, the same shall apply hereinafter). A state immediately thereafter was regarded as an initial state, and the film was subjected to the measurement of the initial moisture content and shrinkage ratio. The initial moisture content was 60% by weight, and the shrinkage ratio was 10%. Then, the film was stored in a desiccator at a temperature of 37°C and a humidity of 90%. After storage for 24 hours, water retention was evaluated as "A" and sufficient water retention was recognized. Immediately after production and after storage for 24 hours, the opposite side could be visually observed through the film in all films, and the films were transparent. Immediately after production and after storage for 24 hours, a change in film shape was not visually observed.

Example 7

The film produced in the same manner as in Example 1, except that dipping in the "ISODINE" (registered trademark) solution was not performed, was dipped in an aqueous 50% by weight glycerin solution for 72 hours. Then, the film was sequentially dipped in three water baths, each containing clean water (200 mL each) therein, for 2 seconds each without a moment's pause, and then surface moisture of the film was slightly wiped off with "Kimwipe". A state immediately thereafter was regarded as an initial state, and the film was subjected to the measurement of the initial moisture content and shrinkage ratio. The initial moisture content was 60% by weight, and the shrinkage ratio was 5%. Using the thus obtained film and an absorbent cotton impregnated with "ISODINE" (register trademark) containing 0.5% by weight of povidone iodine, "ISODINE" (register trademark) was applied on a film surface. Then, the film was stored in a desiccator at a temperature of 37°C and a humidity of 90%. After storage for 24 hours, water retention was evaluated as "A" and sufficient water retention was recognized. The thus obtained film is a satisfactory non-dried film in which an

odor derived from "ISODINE" (register trademark) exists even after the lapse of 48 hours. Immediately after production, after storage for 24 hours and after storage for 48 hours, the opposite side could be visually observed through the film in all films, and the films were transparent. Immediately after production and after storage for 24 hours, a change in film shape was not visually observed.

Example 8

The same test as in Example 6 was carried out, except that an aqueous 10% by weight glycerin solution was used in placed of the aqueous 50% by weight glycerin solution. The initial moisture content was 45% by weight, and the shrinkage ratio was 18%. After storage for 24 hours, water retention was evaluated as "B". Immediately after production and after storage for 24 hours, the opposite side could be visually observed through the film in all films, and the films were transparent. Immediately after production and after storage for 24 hours, a change in film shape was not visually observed.

Comparative Example 1

Using methacrylic acid-2-hydroxyethyl (98 parts by weight), triethylene glycol dimethacrylate (1.0 parts by weight) and a photoinitiator IRGACURE 1850 (1.0 parts by weight), a film was produced and evaluated in the same manner as in Example 1. After storage for 1 day, coloration in water was evaluated as "A" and, after storage for 5 days, coloration in water was evaluated as "C". After storage for 5 days, the film exhibits pale yellow coloration derived from povidone iodine as compared with the film after storage for 1 day and "ISODINE" (register trademark) seep out from the film into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 37% by weight, and the shrinkage ratio was 13.5%.

Comparative Example 2

Using a commercially available soft contact lens containing no silicone component-containing polymer "1-day Acuvue" (register trademark) (constituent monomer: 2-hydroxyethyl methacrylate, methacrylic acid, manufactured by J&J Inc.), the soft contact lens was dipped in an "ISODINE" (registered trademark) solution for 15 minutes and then stored in fresh water for 5 days storage in the same manner as in Example 1. After storage for 1 day, coloration in water was evaluated as "A" and, after storage for 5 days, coloration in water was evaluated as "D". After storage for 5 days, the lens exhibits pale yellow coloration derived from povidone iodine as compared with the lens after storage for 1 day and "ISODINE" (register trademark) seeps out from the lens into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 56.7% by weight, and the shrinkage ratio was 21.6%.

Comparative Example 3

Using a commercially available soft contact lens containing no silicone component-containing polymer "1 day Aquair Evolution" (register trademark) (constituent monomer: 2-hydroxyethyl methacrylate, methacrylic acid, manufactured by The Cooper Companies Inc.), the soft contact lens was dipped in an "ISODINE" (registered trademark) solution for 15 minutes and then stored in fresh water for 5 days storage in the same manner as in Example 1. After storage for 1 day, coloration in water was evaluated as "A" and, after storage for 5 days, coloration in water was evaluated as "D". After storage for 5 days, the lens exhibits pale yellow coloration derived from povidone iodine as compared with the lens after storage for 1 day and "ISODINE" (register trademark) seeps out from the lens into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 51% by weight, and the shrinkage ratio was 19%.

Comparative Example 4

Using a commercially available soft contact lens containing no silicone component-containing polymer "DAILIES" (register trademark) (constituent polymer: modified polyvinyl alcohol, manufactured by CIBA Vision Corporation), the soft contact lens was dipped in an "ISODINE" (registered trademark) solution for 15 minutes and then stored in fresh

water for 5 days storage in the same manner as in Example 1. After storage for 1 day, coloration in water was evaluated as "D" and, after storage for 5 days, coloration in water was evaluated as "D". After storage for 5 days, the lens exhibits pale yellow coloration derived from povidone iodine as compared with the lens after storage for 1 day and "ISODINE" (register trademark) seeps out from the lens into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 70.1% by weight, and the shrinkage ratio was 25.5%.

Comparative Example 5

Using a commercially available soft contact lens containing no silicone component-containing polymer "Medalist" (register trademark) (constituent monomer: 2-hydroxyethyl methacrylate, N-vinylpyrrolidone, manufactured by B&L Inc.), the soft contact lens was dipped in an "ISODINE" (registered trademark) solution for 15 minutes and then stored in fresh water for 5 days storage in the same manner as in Example 1. After storage for 1 day, coloration in water was evaluated as "A" and, after storage for 5 days, coloration in water was evaluated as "C". After storage for 5 days, the lens exhibits pale yellow coloration derived from povidone iodine as compared with the lens after storage for 1 day and "ISODINE" (register trademark) seeps out from the lens into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 55.8% by weight, and the shrinkage ratio was 20.3%.

Example 9

Using a commercially available soft contact lens "$O_2$ OPTIX" (register trademark) (manufactured by CIBA Vision Corporation) produced by using the compounds of the following formulas (j) and (k) in which a silicone monomer includes a polyether structure, the soft contact lens was dipped in an "ISODINE" (registered trademark) solution for 15 minutes and then stored in fresh water for 5 days storage in the same manner as in Example 1. After storage for 1 day, coloration in water was evaluated as "A" and, after storage for 5 days, coloration in water was evaluated as "B". After storage for 5 days, the lens exhibits pale yellow coloration derived from povidone iodine as compared with the lens after storage for 1 day and "ISODINE" (register trademark) seeps out from the lens into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 18.6% by weight, and the shrinkage ratio was 6.8%.

[Chemical Formula 15]

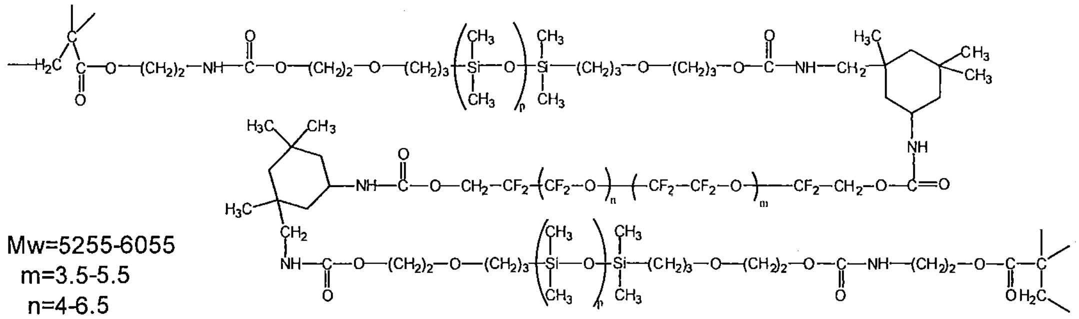

Mw=5255-6055
m=3.5-5.5
n=4-6.5
p=22-26

(j)

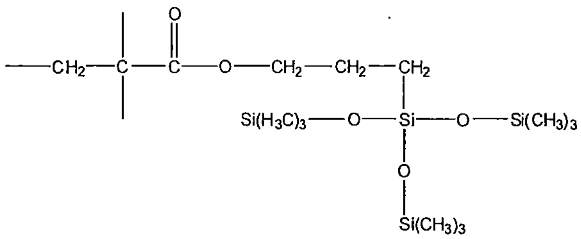

(k)

Example 10

Using a commercially available soft contact lens "2week Premio" (register trademark) (manufactured by Menicon Co., Ltd.) produced by using the compound in which a silicone monomer includes a polyether structure, the soft contact lens was dipped in an "ISODINE" (registered trademark) solution for 15 minutes and then stored in fresh water for 5 days storage in the same manner as in Example 1. After storage for 1 day, coloration in water was evaluated as "A" and, after storage for 5 days, coloration in water was evaluated as "D". After storage for 5 days, the lens exhibits pale yellow coloration derived from povidone iodine as compared with the lens after storage for 1 day and "ISODINE" (register trademark) seeps out from the lens into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the film in all films, and the films were transparent. The initial moisture content was 38.3% by weight, and the shrinkage ratio was 13.9%.

Comparative Example 6

A commercially available gauze "TREX-C" (register trademark) (manufactured by Fuji Systems Corp.), which is an anti-adhesive gauze obtained by coating a gauze made of polyester with a silicone resin (which is polydimethylsiloxane, and is considered to be a silicone component-containing polymer obtained by polymerizing a silicone monomer having a carbon-carbon double bond) was dipped in an "ISODINE" (registered trademark) solution for 15 minutes and then stored in fresh water for 5 days storage in the same manner as in Example 1. After storage for 1 day, coloration in water was evaluated as "D" and, after storage for 5 days, coloration in water was evaluated as "D". After storage for 1 day and after storage for 5 days, pale yellow coloration derived from povidone iodine was not observed, and thus revealing that retention of povidone iodine is inferior as compared with Example 1. The commercially available gauze per se is a white gauze, and the opposite side could not be visually observed through the gauze. The initial moisture content was 0% by weight, and the shrinkage ratio was 0%.

Comparative Example 7

The same test as in Example 6 was carried out, except that water was used in placed of the aqueous 50% by weight glycerin solution. After storage for 24 hours, water retention was evaluated as "C". Immediately after production and after storage for 24 hours, the opposite side could be visually observed through the film in all films, and the films

were transparent. The initial moisture content was 60% by weight, and the shrinkage ratio was 20%. Furthermore, a change in film shape was not visually observed.

Comparative Example 8

The same test as in Example 7 was carried out, except that dipping in the aqueous 50% by weight glycerin solution was not performed. After storage for 24 hours, water retention was evaluated as "C". Immediately after production and after storage for 24 hours, the opposite side could be visually observed through the film in all films, and the films were transparent. The initial moisture content was 52% by weight, and the shrinkage ratio was 22%. Immediately after production and after storage for 24 hours, a change in film shape was not visually observed.

Example 11

A commercially available soft contact lens "$O_2$ OPTIX" (register trademark) (manufactured by CIBA Vision Corporation) produced by using the compound of the following formulas (j) and (k) in which a silicone monomer includes a polyether structure was dipped in an aqueous 50% by weight polyethylene glycol solution for 72 hours and the same test as in Example 6 was carried out. After storage for 24 hours, water retention was evaluated as "C". Immediately after dipping in an aqueous 50% by weight polyethylene glycol solution for 72 hours and after storage for 24 hours, a lens shape became asymmetric due to visual observation and shape retention was inferior as compared with the case before dipping. However, the opposite side could be visually observed through the lens and the lens was transparent. The initial moisture content was 19.9% by weight, and the shrinkage ratio was 5.8%.

Example 12

A commercially available soft contact lens commercially available soft contact lens "2week Premio" (register trademark) (manufacturedby Menicon Co., Ltd.) produced by using the compound in which a silicone monomer includes a polyether structure was dipped in an aqueous 50% by weight polyethylene glycol solution for 72 hours and the same test as in Example 6 was carried out. After storage for 24 hours, water retention was evaluated as "C". Immediately after dipping in an aqueous 50% by weight polyethylene glycol solution for 72 hours and after storage for 24 hours, a lens shape became asymmetric due to visual observation and shape retention was inferior as compared with the case before dipping. However, the opposite side could be visually observed through the lens and the lens was transparent. The initial moisture content was 40.2% by weight, and the shrinkage ratio was 11.9%.

Comparative Example 9

Using a commercially available silicone film (product number 6-9085-02, thickness 0.1 mm) (manufactured by AS ONE Corporation) produced by using the compound in which a silicone monomer does not include a carbon-carbon double bond and a polyether structure, the silicone film was dipped in an "ISODINE" (registered trademark) solution for 15 minutes and then stored in fresh water for 5 days storage in the same manner as in Example 1. After storage for 1 day, coloration in water was evaluated as "E" and, after storage for 5 days, coloration in water was evaluated as "E". After storage for 1 day and after storage for 5 days, coloration derived from povidone iodine was not observed, and thus revealing that retention of povidone iodine is inferior as compared with Example 1. The commercially available silicone film per se is a semitransparent white film, and was not transparent enough to enable the opposite side to be visually observed through the film. The initial moisture content was 0% by weight, and the shrinkage ratio was 0%.

Comparative Example 10

By the method disclosed in Example 1 of Japanese Unexamined Patent Publication (Kokai) No. 2009-148392, a wound coating material made of a polyether-based polymer in which a skin contact layer is made of a polyether-based polymer having a siloxane bond (which is not a silicone component-containing polymer obtained by polymerizing a silicone monomer having a carbon-carbon double bond) was produced. The wound coating material was dipped in an "ISODINE" (registered trademark) solution for 15 minutes and then stored in fresh water for 5 days in the same manner as in Example 1. After storage for 1 day, coloration in water was evaluated as "A" and, after storage for 5 days, coloration in water was evaluated as "C". After storage for 5 days, the skin contact layer exhibits pale yellow coloration derived from povidone iodine as compared with the film after storage for 1 day and "ISODINE" (register trademark) seeps out from the film into water, and thus revealing that retention of povidone iodine is inferior as compared with Example 1. Immediately after dipping in an "ISODINE" (registered trademark) solution, after storage for 1 day and after storage for 5 days, the opposite side could be visually observed through the skin contact layer in all skin contact layers, and the

skin contact layer was transparent. The initial moisture content of the skin contact layer was 40% by weight, and the shrinkage ratio was 14.5%.

Comparative Example 11

The wound coating material produced in Comparative Example 10 was dipped in an aqueous 50% by weight glycerin solution for 72 hours (glycerin has a boiling point of 290°C) . Then, the wound coating material was sequentially dipped in three water baths, each containing clean water (200 mL each), for 2 seconds each without a moment's pause, and then surface moisture of the wound coating material was slightly wiped off with "Kimwipe". A state immediately thereafter was regarded as an initial state, and the film was subjected to the measurement of the initial moisture content and shrinkage ratio. Then, the thus obtained film was stored in a desiccator at a temperature of 37°C and a humidity of 90%. After storage for 24 hours, water retention was evaluated as "C". Immediately after dipping in an aqueous 50% by weight glycerin solution for 72 hours and after storage for 24 hours, a shape of the skin contact layer became asymmetric due to visual observation and shape retention was inferior as compared with the case before dipping. However, the opposite side could be visually observed through the skin contact layer, and the skin contact layer was transparent. The initial moisture content was 42% by weight, and the shrinkage ratio was 15.3%.

With respect to the Examples and Comparative Examples, coloration evaluation results are collectively shown in Table 1, and water retention evaluation results are collectively shown in Table 2.

[table 1] only examples 1-3 are according to the invention.

| | Evaluation results of coloration | |
|---|---|---|
| | After storage for 1 day | After storage for 5 days |
| Example 1 | A | A |
| Example 2 | A | A |
| Example 3 | A | A |
| Example 4 | A | A |
| Example 5 | A | A |
| Comparative Example 1 | A | C |
| Comparative Example 2 | A | D |
| Comparative Example 3 | A | D |
| Comparative Example 4 | D | D |
| Comparative Example 5 | A | C |
| Example 9 | A | B |
| Example 10 | A | D |
| Comparative Example 6 | D | D |
| Comparative Example 9 | E | E |
| Comparative Example 10 | A | C |

[table 2] - only example 7 is according to the invention.

| | Evaluation results of water retention |
|---|---|
| Example 6 | A |
| Example 7 | A |
| Example 8 | B |
| Comparative Example 7 | C |
| Comparative Example 8 | C |

(continued)

| | Evaluation results of water retention |
|---|---|
| Example 11 | C |
| Example 12 | C |
| Comparative Example 11 | C |

<Evaluation 1 of Antibacterial Properties>

Evaluation of antibacterial properties of materials for skin obtained in Examples 1 to 3, Comparative Example 1 and Comparative Example 6 was carried out. Antibacterial properties were evaluated by the following test method. Before dipping the sheet-like film produced by the above method in an "ISODINE" (registered trademark) solution, the film was cut into pieces measuring 1 cm square and the film was dipped in an "ISODINE" (registered trademark) solution (povidone iodine concentration 7%, manufactured by Meiji Seika Kaisha, Limited) for 15 minutes in the same manner as in Example 1. Then, the film was washed with water and stored in water for 5 days. After the storage, the film was taken out from water and surface moisture was slightly wiped off, and then saprophytic bacteria were adhered by touching with an index finger. This film was placed on a Trypto-Soya agar medium (SCD agar, manufactured by NIHON PHARMACEUTICAL CO., LTD.) and stored in an incubator at 37°C for 5 days storage. A control film was produced in the same manner, except that dipping in an "ISODINE" (registered trademark) solution was not performed. Antibacterial properties were evaluated there times per one kind of a sample. A state of the development of bacteria developed on an agar medium was observed. The case where, the area where bacteria have developed three times accounts for 10% or less of the area of the entire film, was judged that antibacterial activity exists. The area is more preferably 5% or less, and most preferably 0%. The results are as shown in Table 3. The area where bacteria have developed of a control film, which was not dipped in an "ISODINE" (registered trademark), was 100%, whereas, the area where bacteria have developed of the films produced in Examples 1 to 3 was 0%, and the films exhibited excellent antibacterial properties. In contrast, the films produced in Comparative Example 1 and Comparative Example 6 did not exhibit antibacterial properties.

[Table 3]

| | Area where bacteria have been developed after culturing for 5 days | |
|---|---|---|
| | Dipped in ISODINE | Not dipped in ISODINE |
| Example 1 | 0% | 100% |
| Example 2 | 0% | 100% |
| Example 3 | 0% | 100% |
| Comparative Example 1 | 80% | 100% |
| Comparative Example 6 | 100% | 100% |

<Evaluation 2 of Antibacterial Properties>

Evaluation of antibacterial properties of Examples 6 and 7 was carried out. In the same manner as in the above-mentioned <Evaluation 1 of Antibacterial Properties>, a comparison is made between antibacterial properties of the film of Example 6 in which an "ISODINE" (registered trademark) solution is not applied, and antibacterial properties of the film of Example 7 in which an "ISODINE" (registered trademark) solution was applied. The results are as shown in Table 4. The area where bacteria have developed of the film of Example 6 was 100%, whereas, the area where bacteria have developed of the film of Example 7 was 10% and the film exhibited antibacterial properties.

[Table 4]

| | Area where bacteria have been developed after culturing for 1 day |
|---|---|
| Example 6 (not dipped in ISODINE) | 100% |
| Example 7 (dipped in ISODINE) | 10% |

[Industrial Applicability]

The material for skin of the present invention for skin is suited for use in various applications, for example, skin protective materials for the purpose of coating or preventing wound, inflammation, bedsore and the like; sanitary goods such as sanitray items and paper diapers; various drug carriers such as medicines for external use (base material for applying to the skin); cosmetics such as materials for gel-like face mask; and cell culture media (cell proliferation media) . The material for skin is particularly suited for use in skin protective materials.

## Claims

1. A material for skin, comprising a silicone component-containing polymer obtained by polymerizing a silicone monomer having a carbon-carbon double bond, and an antibacterial agent,
   wherein the silicone monomer is a silicone monomer including no polyether structure,
   wherein the antibacterial agent contains halogen, and
   wherein the content of hydroxyl groups in the silicone monomer is from 0.0005 to 0.01 equivalent/g.

2. The material for skin according to claim 1, wherein the material for skin is a sheet.

3. The material for skin according to claim 2, wherein the material for skin has an initial moisture content of 10 to 80% by weight, and also has a shrinkage ratio of 20% or less.

4. The material for skin according to any one of claims 1 to 3, further comprising an alcohol having a boiling point of 150°C or higher.

5. The material for skin according to any one of claims 1 to 4, wherein the content of silicon atoms in the material for skin is from 5 to 30% by weight based on the dry weight of the material for skin.

6. The material for skin according to any one of claims 1 to 5, wherein the application of the material for skin is at least one selected from a sanitary good, a cosmetic, a cell culture medium, a skin protective material and a drug carrier.

7. A method for producing a material for skin, which comprises imparting an antibacterial agent to a silicone component-containing polymer obtained by polymerizing a silicone monomer having a carbon-carbon double bond,
   wherein the silicone monomer is a silicone monomer including no polyether structure,
   wherein the antibacterial agent contains halogen,
   wherein the content of hydroxyl groups in the silicone monomer is from 0.0005 to 0.01 equivalent/g, and
   wherein a silicone component-containing polymer obtained by polymerizing a silicone monomer composition having a carbon-carbon double bond is dipped in a solution containing an antibacterial agent.

## Patentansprüche

1. Material für die Haut, umfassend ein Silikonkomponenten-enthaltendes Polymer, das durch Polymerisieren eines Silikonmonomers mit einer Kohlenstoff-Kohlenstoff-Doppelbindung erhalten wird, und ein antibakterielles Mittel,
   wobei das Silikonmonomer ein Silikonmonomer ist, das keine Polyetherstruktur enthält,
   wobei das antibakterielle Mittel Halogen enthält, und
   wobei der Gehalt an Hydroxylgruppen im Silikonmonomer 0,0005 bis 0,01 Äquivalent/g beträgt.

2. Material für die Haut nach Anspruch 1, wobei das Material für die Haut ein Blatt ist.

3. Material für die Haut nach Anspruch 2, wobei das Material für die Haut einen anfänglichen Feuchtigkeitsgehalt von 10 bis 80 Gew. -% aufweist und auch ein Schrumpfungsverhältnis von 20 % oder weniger aufweist.

4. Material für die Haut nach einem der Ansprüche 1 bis 3, ferner umfassend einen Alkohol mit einem Siedepunkt von 150 °C oder höher.

5. Material für die Haut nach einem der Ansprüche 1 bis 4, wobei der Gehalt an Siliziumatomen in dem Material für die Haut 5 bis 30 Gew.-%, bezogen auf das Trockengewicht des Materials für die Haut, beträgt.

6. Material für die Haut nach einem der Ansprüche 1 bis 5, wobei die Anwendung des Materials für die Haut mindestens eines ausgewählt aus einem Hygieneartikel, einer Kosmetik, einem Zellkulturmedium, einem Hautschutzmaterial und einem Wirkstoffträger ist.

7. Verfahren zur Herstellung eines Materials für die Haut, umfassend das Versetzen eines Silikonkomponenten-enthaltenden Polymers, das durch Polymerisation eines Silikonmonomers mit einer Kohlenstoff-Kohlenstoff-Doppelbindung erhalten wird, mit einem antibakteriellen Mittel,
wobei das Silikonmonomer ein Silikonmonomer ist, das keine Polyetherstruktur enthält,
wobei das antibakterielle Mittel Halogen enthält,
wobei der Gehalt an Hydroxylgruppen im Silikonmonomer 0,0005 bis 0,01 Äquivalent/g beträgt, und
wobei ein Silikonkomponenten-enthaltendes Polymer, das durch Polymerisation einer Silikonmonomerzusammensetzung mit einer Kohlenstoff-Kohlenstoff-Doppelbindung erhalten wird, in eine Lösung getaucht wird, die ein antibakterielles Mittel enthält.

## Revendications

1. Matière pour la peau, comprenant un polymère contenant un composant de silicone obtenu par la polymérisation d'un monomère de silicone ayant une liaison double carbone-carbone, et un agent antibactérien,
dans laquelle le monomère de silicone est un monomère de silicone n'incluant aucune structure de polyéther,
dans laquelle l'agent antibactérien contient un halogène, et
dans laquelle la teneur en groupes hydroxyle dans le monomère de silicone est de 0,0005 à 0,01 équivalent/g.

2. Matière pour la peau selon la revendication 1,
dans laquelle la matière pour la peau est une feuille.

3. Matière pour la peau selon la revendication 2,
dans laquelle la matière pour la peau présente une teneur initiale en humidité de 10 à 80 % en poids, et présente également un taux de retrait de 20 % ou moins.

4. Matière pour la peau selon l'une quelconque des revendications 1 à 3, comprenant en outre un alcool ayant un point d'ébullition de 150 °C ou plus.

5. Matière pour la peau selon l'une quelconque des revendications 1 à 4, dans laquelle la teneur en atomes de silicium dans la matière pour la peau est de 5 à 30 % en poids sur la base du poids sec de la matière pour la peau.

6. Matière pour la peau selon l'une quelconque des revendications 1 à 5, dans laquelle l'application de la matière pour la peau est au moins l'un sélectionné parmi un produit sanitaire, un produit cosmétique, un milieu de culture de cellules, une matière protectrice de la peau et un support pour médicament.

7. Procédé pour produire une matière pour la peau, qui comprend le fait de conférer un agent antibactérien à un polymère contenant un composant de silicone obtenu par la polymérisation d'un monomère de silicone ayant une liaison double carbone-carbone,
dans lequel le monomère de silicone est un monomère de silicone n'incluant aucune structure de polyéther,
dans lequel l'agent antibactérien contient un halogène,
dans lequel la teneur en groupes hydroxyle dans le monomère de silicone est de 0,0005 à 0,01 équivalent/g, et
dans lequel un polymère contenant un composant de silicone obtenu par la polymérisation d'une composition de monomère de silicone ayant une liaison double carbone-carbone est immergé dans une solution contenant un agent antibactérien.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2007129474 A1
- JP 2002104974 A
- WO 2002022182 A
- JP 2009148392 A
- JP 2009148393 A
- US 20080081894 A